# EUROPEAN PATENT APPLICATION

(11) **EP 1 842 913 A1**
(43) Date of publication of application: **10.10.2007**
(21) Application number: 06122123.0
(22) Date of filing: 11.10.2006
(51) Int. Cl.: C12N 15/09

(54) **Method and apparatus for purifying nucleic acid on hydrophilic surface of solid support using hydrogen bonding**

(30) Priority: 06.04.2006 KR 20060031490
(71) Applicant: Samsung Electronics Co., Ltd., Suwon-si, Gyeonggi-Do (KR)
(72) Inventor: Lee, In-ho, Gyeonggi-do (KR); Yoo, Chang-eun, Seoul (KR); Han, Ki-woong, Seoul (KR); Kim, Young-rok, Gyeonggi-do (KR); Min, Jun-hong, Gyeonggi-do (KR)
(74) Representative: Grünecker, Kinkeldey, Stockmair & Schwanhäusser Anwaltssozietät

(57) **Abstract**

Provided is a method of purifying nucleic acid, the method including:
contacting a nucleic acid-containing sample and a solution containing a kosmotropic salt on a solid support having a hydrophilic functional group on its surface to bind the nucleic acid to the solid support. Since the solid support is used as it is without any surface treatment, manufacture of the apparatus is very easy, and nucleic acid can be bound to the solid support without specific additives in a wide pH range, so that the apparatus can be used for a Lab-On-a-Chip.

## Description

### BACKGROUND OF THE INVENTION

### 1. Field of the Invention

The present invention relates to a method and apparatus for purifying nucleic acid on a hydrophilic surface of a solid support using hydrogen bonding.

### 2. Description of the Related Art

Methods of isolating DNA from cells are performed using materials that have the proclivity of binding to DNA. Examples of materials used for isolating DNA include silica, glass fiber, anion exchange resin and magnetic beads (Rudi, K. et al., Biotechniqures 22, 506-511 (1997); and Deggerdal, A. et al., Biotechniqures 22, 554-557 (1997)). To avoid manual operation and to remove operator error, automatic machines have been developed for high-throughput DNA extraction.

The production of high purity double-stranded plasmid DNAs, single-stranded phage DNAs, chromosomal DNAs, and agarose gel-purified DNA fragments is very important in molecular biology. Ideal methods of purifying DNAs should be simple and quick, and include little additional manipulation of samples. The DNAs obtained using such methods can be used for direct transformation, restriction enzyme analysis, ligation, or sequencing. Such methods are very attractive in the automated production of DNA samples, which is favored in research and diagnosis labs.

Conventionally, a method of purifying nucleic acid using a solid phase is known. For example, U.S. Patent No. 5,234,809 discloses a method of purifying nucleic acid using a solid phase to which nucleic acids are bound, the method including: mixing a starting material, a chaotropic material, and a nucleic acid binding solid phase; separating the solid phase with the nucleic acid bound thereto from the liquid, and washing the solid phase nucleic acid complexes. However, this method is time consuming and complicated, and thus is not suitable for a Lab-On-a-Chip (LOC). The method also has a problem in that the chaotropic material should be used.

U.S. Patent No. 6,291,166 discloses a method of archiving nucleic acid using a solid-phase matrix. This method is advantageous in that since nucleic acids are irreversibly bound to the solid-phase matrix, delayed analysis or repeated analysis of the nucleic acid solid-phase matrix complexes is possible. However, according to this method, alumina, which has a positively charged surface, needs to be rendered hydrophilic by addition of basic materials, such as NaOH, and nucleic acids are irreversibly bound to the hydrophilic alumina, and thus cannot be separated from the alumina.

U.S. Patent No. 6,383,783 discloses a method of isolating nucleic acid from a sample, the method including: employing a sample containing target nucleic acids on a hydrophobic organic polymer solid-phase in order to attach target nucleic acid on a solid-phase; and adding a non-ionic surfactant to the solid-phase and removing the attached target nucleic acid. This phrase is not commonly used in this section of a specification in that the invention disclosed in U.S. Patent No. 6,383,783 uses a hydrophilic solid-phase, but the present invention uses a solid-phase having a hydrophilic surface and kosmotropic salts.

The inventors of the present invention studied a method of purifying nucleic acid based on the prior art as described above, and confirmed that when kosmotropic salts are added onto a solid support having a hydrophilic functional group on its surface, nucleic acids can be bound thereto regardless of pH.

### SUMMARY OF THE INVENTION

The present invention provides a method of purifying nucleic acid using a solid support having a hydrophilic surface, the method including: contacting a nucleic acid-containing sample and a solution containing a kosmotropic salt on a solid support having a hydrophilic functional group on its surface to bind the nucleic acid to the solid support.

The present invention also provides an apparatus for purifying nucleic acid, the apparatus including: a solid support having a hydrophilic functional group on its surface; and a kosmotropic salt solution storing part that is interconnected to the solid support through a microchannel and provides the kosmotropic salt to the solid support.

The present invention also provides a lab-on-a-chip including the apparatus for purifying nucleic acid.

### BRIEF DESCRIPTION OF THE DRAWINGS

The above and other features and advantages of the present invention will become more apparent by describing in detail exemplary embodiments thereof with reference to the attached drawings in which:
FIG. 1 is a schematic view showing that nucleic acid is bound to a hydrophilic surface of a solid support in the presence of a kosmotropic salt;
FIG. 2 is a graph showing the binding efficiency of E. coli gDNA that is bound to a silica substrate according to pH;
FIG. 3 is a graph showing the binding efficiency of E. coli gDNA according to a pH and concentration of a kosmotropic salt and chaotropic salt;
FIG. 4 is a graph showing the binding efficiency of E. coli gDNA according to types of a substrate surface; and
FIG. 5 is a graph showing the binding and eluting efficiency of E. coli gDNA using a kosmotropic salt.

### DETAILED DESCRIPTION OF THE INVENTION

The present invention will now be described more fully with reference to the accompanying drawings, in which exemplary embodiments of the invention are shown.

The present invention provides a method of purifying nucleic acid using a solid support which has a hydrophilic functional group on its surface, the method including: contacting a nucleic acid-containing sample and a solution comprising a kosmotropic salt on the solid support to bind the nucleic acid to the solid support.

In the method according to the present invention, nucleic acids are bound to a solid support surface having a hydrophilic functional group regardless of pH using a kosmotropic salt solution, and a low salt solution is added thereto to elute nucleic acids. In a conventional method of isolating nucleic acid a chaotropic salt should be used, pH should be adjusted, a solid support surface should be modified, or extra specific additives such as polyethylene glycol(PEG) should be added. In a method according to an embodiment of the present invention, there is little limitation on surface conditions of a solid support, and nucleic acids can be bound to a solid support at a wide pH range without specific additives.

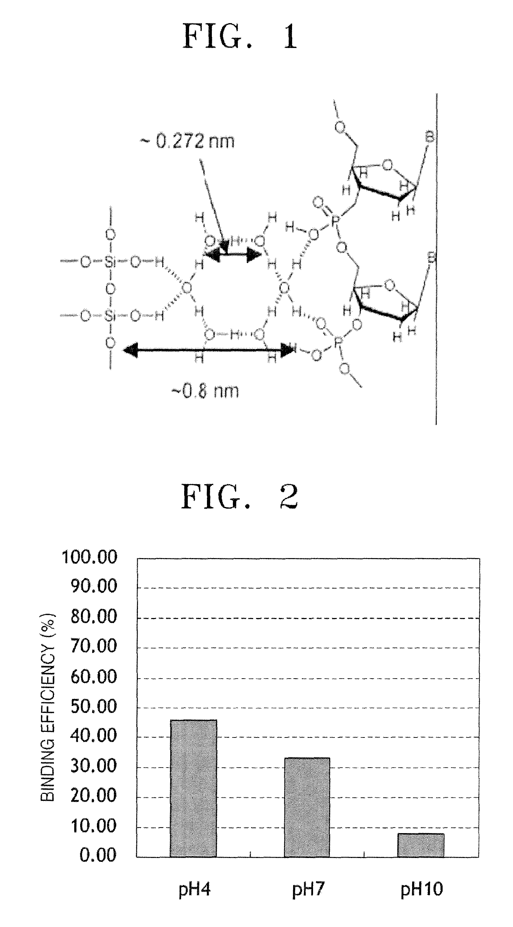

According to an embodiment of the present invention, a kosmotropic salt is used to bind nucleic acids to a solid support. In the case of a chaotropic salt, a solid support surface is dehydrated and nucleic acids are directly bound to the solid support by hydrogen bonding, so that the pH of the solid support is important. However, in the case of a kosmotropic salt, the solid support surface is hydrated so that a stable water layer is formed thereon. Nucleic acid is considered to be hydrated on the hydrated solid support surface, and it stems from a salting-out effect by hydrophilic interaction. FIG. 1 is a schematic view showing that nucleic acid is bound to the hydrophilic surface of a solid support in the presence of a kosmotropic salt. Referring to FIG. 1, water forms hydrogen bonds on a silica substrate (on a left side of FIG. 1) due to a salting out effect of the kosmotropic salt, and the formed water layer (in a middle portion of FIG. 1) forms hydrogen bonds with nucleic acid (on a right side of FIG. 1) again, so that nucleic acid is bound to the solid support by means of the stable water layer.

Therefore, nucleic acid binding with a solid support does not require an acidic condition that is generally required for nucleic acid binding, and the solid support surface needs not be limited to silica.

Examples of a hydrophilic functional group on a solid support include a hydroxyl group, an amine group, a carboxyl group, a polycarboxyl group and the like, but are not limited thereto.

In the method according to the current embodiment of the present invention, the method further comprises washing a sample that is not bound to the solid support after the nucleic acid binding. Through this process, nucleic acid is bound to the solid support, and then a sample that is not bound to the solid support is washed, so that nucleic acid can be purified in a purer form. A solution used in nucleic acid binding with the solid support can be used as a washing solution.

In the method according to the current embodiment of the present invention, the method further comprises eluting the nucleic acid bound to the solid support by adding water or a nucleic acid eluting buffer solution. Nucleic acid itself that is bound to the solid support can be used, but eluting the nucleic acid bound to the solid support is required for the more efficient use of the bound nucleic acid, for example, amplification or detection of isolated nucleic acid.

In the method according to the current embodiment of the present invention, the kosmotropic salt can be sulfate(SO₄²⁻), phosphate(HPO₄²⁻), hydroxide(OH⁻), fluoride(F⁻), formate(HCOO⁻), acetate(CH₃COO⁻) or the like, but is not limited thereto. The kosmotropic salt induces crystallization of proteins, functions as a salting-out ion for hydrophobic particles, and forms a water structure, according to the Hofmeister series.

In the method according to the current embodiment of the present invention, the nucleic acid-containing sample and a solution containing a kosmotropic salt may have a pH of 3-10. When the pH of the nucleic acid-containing sample and a solution containing a kosmotropic salt is beyond this range, DNA can be physically and chemically denatured, and thus subsequent processes can be affected.

In the method according to the current embodiment of the present invention, the concentration of the kosmotropic salt may be 100-2,000 mM. When the concentration of the kosmotropic salt is less than 100 mM, binding efficiency of nucleic acid that is bound to the solid support decreases. When the concentration of the kosmotropic salt is greater than 2,000 mM, it is difficult to prepare the solution.

In the method according to the current embodiment of the present invention, the solid support can be a slide glass, a silicon wafer, a magnetic bead, polystyrene, a membrane, a metal plate, or the like, but is not limited thereto. The solid support can be any material having a hydrophilic functional group on its surface, but it should be insoluble in water. When the solid support is soluble in water, it is difficult to separate a nucleic acid solution from the solid support after the nucleic acid is purified. In addition, use of a solid support having a large surface area is desirable because more hydrophilic functional groups can be included on its surface. Therefore, in the case of a planar solid support, such as glass or a wafer, the surface of the planar solid support is processed to have a pillar structure to increase its surface area.

In the method according to the current embodiment of the present invention, the nucleic acid eluting buffer can be phosphate, Tris, HEPES, CHES, borate or the like, but is not limited thereto.

In the method according to the current embodiment of the present invention, the nucleic acid eluting buffer may have a pH of 5-10. When the pH of the nucleic acid eluting buffer is less than pH 5, eluting efficiency of the nucleic acid bound to a solid support decreases. When the pH of the nucleic acid eluting buffer is greater than pH 10, subsequent processes may be affected.

In the method according to the current embodiment of the present invention, the concentration of the nucleic acid eluting buffer may be less than or equal to 100 mM. When the concentration of the nucleic acid eluting buffer is greater than 100 mM, eluting efficiency of the nucleic acid bound to a solid support decreases and also subsequent processes may be affected. If the nucleic acid eluting buffer is water, the concentration of the nucleic acid eluting buffer is 0 mM.

In the method according to the current embodiment of the present invention, the binding of the nucleic acid to the solid support or the eluting of the nucleic acid from the solid support can be performed in a static or fluidic state. Contacting of the nucleic acid and the solid support can be performed both in a static state and in a fluidic state. That is, the solid support is contacted with the nucleic acid while a solution containing the nucleic acid flows in a flow control system. In the flow control system, the solid support can be planar. However, in order to contact more nucleic acid with the solid support, the solid support can have a large amount of pillar structures.

In the method according to the current embodiment of the present invention, the sample containing the nucleic acid can be blood, serum, urine, saliva, ocular lens fluid, cerebrospinal fluid, milk, ascite fluid, synovial fluid, peritoneal cavity liquid, amniotic fluid, tissue, fermentation broth, cell culture fluid, nucleic acid amplification reaction product, nucleic acid synthesis product, or the like, but is not limited thereto. The sample containing the nucleic acid according to an embodiment of the present invention may be from a mammal, a plant, bacteria, or yeast. The sample can be in a single cell form or tissue form, and the cell or tissue may stem from cultures in vitro.

The nucleic acid purified using the method according to the current embodiment of the present invention may have any molecular weight, and have a single-stranded form, a double-stranded form, a circular form, a plasmid form or the like. For example, nucleic acids, such as small oligonucleotide or nucleic acid molecule of nucleotide having a length of about 10-50, a longer molecule of nucleotide having a length of about 1,000-10,000, nucleic acid having even a larger molecular weight of about 50 kb-500 kb can be isolated using the method according to the current embodiment of the present invention.

In the method according to the current embodiment of the present invention, the method further comprises detecting the nucleic acid eluted after eluting nucleic acid from the solid support. The eluted nucleic acid can be detected by electrophoresis, sequencing or the like.

In the method according to the current embodiment of the present invention, the method further comprises amplifying the nucleic acid eluted after eluting nucleic acid from the solid support. When the nucleic acid cannot be directly detected due to a very small amount of the eluted nucleic acid, the nucleic acid can be easily detected by amplifying the eluted nucleic acid using a PCR method.

In the method according to the current embodiment of the present invention, amplifying the nucleic acid can be performed without removing a nucleic acid eluting buffer. The nucleic acid eluting buffer has almost the same composition as the buffer used in nucleic acid amplification, so that the nucleic acid eluted in the nucleic acid eluting buffer can be immediately amplified without removing the nucleic acid eluting buffer.

The present invention also provides an apparatus for purifying nucleic acid includes: a solid support having a hydrophilic functional group on its surface; and a kosmotropic salt solution storing part that is interconnected to the solid support through a microchannel and provides a kosmotropic salt to the solid support.

The apparatus for purifying nucleic acid according to the current embodiment of the present invention essentially comprises a kosmotropic salt solution storing part and a solid support having a hydrophilic functional group on its surface. The kosmotropic salt solution storing part is a part that provides the kosmotropic salt to the solid support, and is interconnected to the solid support through a microchannel. When a sample containing nucleic acid to be isolated is introduced into the apparatus, the kosmotropic salt in the kosmotropic salt solution storing part is provided to the solid support, the sample containing nucleic acid and kosmotropic salt are mixed in the solid support, and then the nucleic acid is bound to the solid support by the salting-out effect of the kosmotropic salt. To elute the bound nucleic acid, the apparatus for purifying nucleic acid according to the current embodiment of the present invention further comprises a nucleic acid eluting buffer storing part that is interconnected to the solid support through a microchannel and provides a nucleic acid eluting buffer to the solid support.

In the apparatus according to the current embodiment of the present invention, the solid support can have a planar structure, a pillar structure, a bead structure or a sieve structure, but is not limited thereto.

In the apparatus according to the current embodiment of the present invention, the solid support can be slide glass, a silicon wafer, a magnetic bead, polystyrene, a membrane, a metal plate or the like, but is not limited thereto. The solid support can be any material having a hydrophilic functional group on its surface, but should be insoluble in water. When the solid support is soluble in water, it is difficult to separate a nucleic acid solution from the solid support after the nucleic acid is purified. In addition, use of a solid support having a large surface area is desirable because more hydrophilic functional groups can be included on its surface. Therefore, in the case of a planar solid support, such as glass or a wafer, the surface of the planar solid support is processed to have a pillar structure to increase its surface area.

In the apparatus according to the current embodiment of the present invention, the kosmotropic salt that is stored in a kosmotropic salt solution storing part and introduced to a solid support through a micro channel can be sulfate (SO₄²⁻), phosphate(HPO₄²⁻), hydroxide(OH⁻), fluoride(F-), formate(HCOO⁻), acetate(CH₃COO⁻) or the like, but is not limited thereto.

In the apparatus according to the current embodiment of the present invention, the apparatus further comprises a nucleic acid amplification part or detection part that can amplify or detect the nucleic acid eluted after eluting nucleic acid from the solid support. In the nucleic acid amplification part, when the nucleic acid cannot be directly detected due to a very small amount of the eluted nucleic acid, the eluted nucleic acid can be amplified using a PCR device. In the nucleic acid detection part, an electrophoresis device, a sequencing device or the like can be used in order to see whether eluted nucleic acid exists.

According to another embodiment of the present invention, there is provided a lab-on-a-chip including the apparatus for purifying nucleic acid. In the apparatus for purifying nucleic acid according to an embodiment of the present invention, each functional element can be implemented by process-on-a-chip using a known microfluidic technique and a MEMS device, and can be further implemented by a lab-on-a-chip.

Hereinafter, the present invention will be described in further detail with reference to the following examples. These examples are for illustrative purposes only and are not intended to limit the scope of the present invention.

### Comparative example 1: Binding efficiency of nucleic acid according to pH using a Qiagen DNA purification system

Binding efficiency of nucleic acid according to pH using a Qiagen DNA purification system was determined. Using E. coli lysate including E. coli BL21 gDNA 4688ng as a sample including nucleic acid and using a Qiagen kit(Cat. # 51306) having a silica surface as a substrate surface, binding efficiency of nucleic acid was measured at pH 4, 7 and 10.

FIG. 2 is a graph showing binding efficiency of E. coli gDNA that is bound to the silica substrate according to pH. As can be seen in FIG. 2, the nucleic acid binding efficiency is the highest at pH 4, and the nucleic acid binding efficiency is the lowest at pH 10. Therefore, it can be seen that binding efficiency of nucleic acid is significantly reduced as pH increases. This is because when a pH of a solution increases, negative charges on the substrate surface increase, and an electrostatic repulsive force between the increased negative charges and DNA having negative charges increases, so that DNA binding efficiency decreases.

Therefore, nucleic acid binding is possible at a low pH in the Qiagen DNA purification system that does not use a kosmotropic salt, but nucleic acid binding efficiency is significantly reduced at a high pH, and thus it can be seen that nucleic acid is highly affected by pH while isolating nucleic acid.

### Example 1: Nucleic acid binding efficiency using the method according to an embodiment of the present invention

Binding efficiency of nucleic acid using the method according to an embodiment of the present invention was determined. The experiment was performed in the same manner as in Comparative Example 1, except that SO₄²⁻ was used as a kosmotropic salt, SCN⁻ was used as a chaotropic salt, and 0, 10, 1,000 and 2,000 mM of SO₄²⁻ and SCN⁻, respectively were used, and nucleic acid binding was performed at pH 4, 6.5-7.5 and 10.

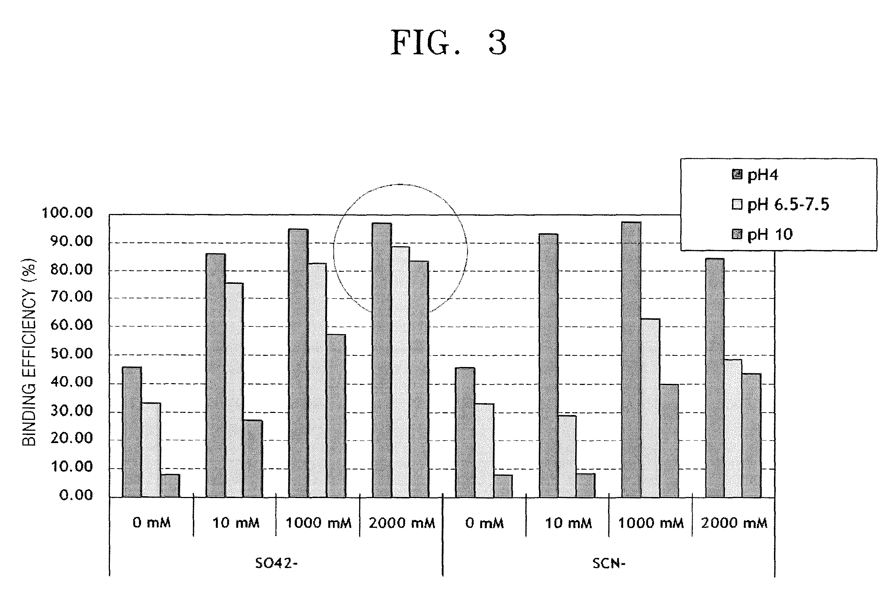

FIG. 3 is a graph showing binding efficiency of E. coli gDNA according to pH and concentration of a kosmotropic salt or chaotropic salt. In FIG. 3, a left panel represents results of measuring binding efficiency of E. coli gDNA using SO₄²⁻ as a kosmotropic salt, and a right panel represents results of measuring binding efficiency of E. coli gDNA using SCN⁻ as a chaotropic salt. As can be seen in FIG. 3, generally, the nucleic acid binding efficiency is the highest at pH 4, and the nucleic acid binding efficiency is the lowest at pH 10, and it can be seen that nucleic acid binding efficiency increases as a concentration of the salts increases. However, unlike Comparative Example 1, in the case of using a kosmotropic salt, as a concentration of SO₄²⁻ increases, binding efficiency of nucleic acid increases even at pH 10. Therefore, it is seen that binding efficiency of nucleic acid has little difference at pH 4 and pH 10 when a concentration of SO₄²⁻ is 2000 mM. However, when a chaotropic salt is used in a low concentration, binding efficiency of nucleic acid is similar to that when a kosmotropic salt is used in a low concentration. On the other hand, when a concentration of the salt is 2,000 mM, binding efficiency of nucleic acid decreases as pH increases, unlike a kosmotropic salt.

The results described above stem from a water hydration effect, and the kosmotropic salt hydrates a substrate surface, so that it enhances a water network, and the strong network of water layer on the silica surface plays a critical role when DNA is bound to the surface by hydrogen bonding.

Therefore, when binding nucleic acid to the surface using a kosmotropic salt according to an embodiment of the present invention, nucleic acid can be efficiently bound regardless of pH by addition of a proper amount of the kosmotropic salt.

### Example 2: Binding efficiency of nucleic acid according to substrate surface types

Binding efficiency of nucleic acid according to different types of a substrate surface was determined. The substrate was a glass bead, a glass bead having polycarboxyl terminal or a glass bead having carboxyl terminal. The experiment was performed in the same manner as in Comparative Example 1, except that 1 M of sodium sulfate(pH 4, pH 7) was used as a kosmotropic salt, and E. coli gDNA 1515ng was used as nucleic acid.

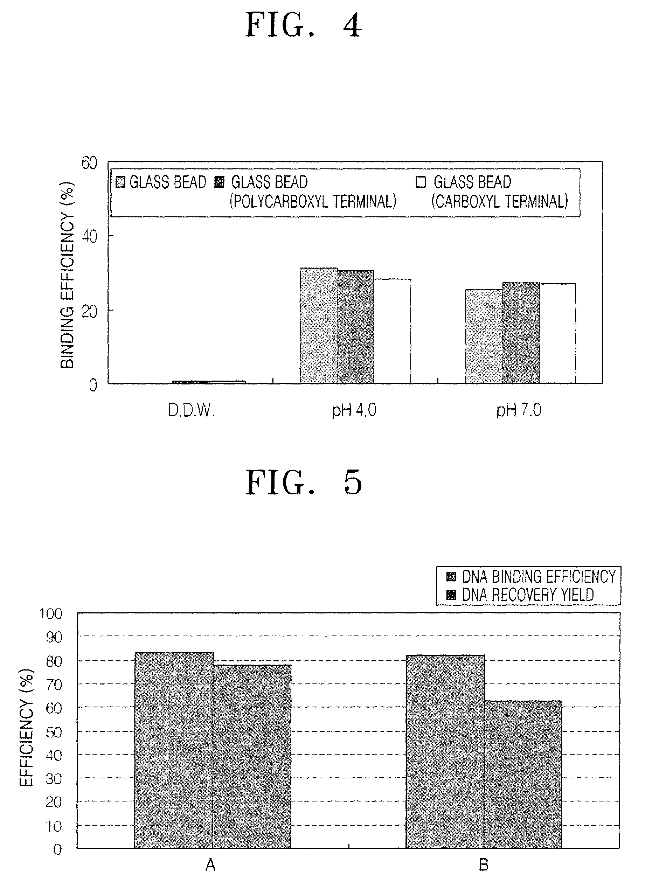

FIG. 4 is a graph showing binding efficiency of E. coli gDNA according to types of a substrate surface. In FIG. 4, results represented by DDW refer to results when distilled water was used in the absence of a kosmotropic salt, and results at pH 4.0 and pH 7.0 refer to results when 1 M of sodium sulfate as a kosmotropic salt was added. As can be seen in FIG. 4, while binding efficiency of nucleic acid is very low when a kosmotropic salt is not added, binding efficiency of nucleic acid is significantly increased when a kosmotropic salt is added. In addition, binding efficiency of nucleic acid at pH 4.0 and pH 7.0 shows little change according to the substrate surface types.

Therefore, when a kosmotropic salt is added while binding nucleic acid to a substrate, nucleic acid is efficiently bound to the substrate regardless of pH or the substrate surface types.

### Example 3: Eluting efficiency of nucleic acid using the method according to an embodiment of the present invention

Eluting efficiency of nucleic acid that was bound to a substrate using the method according to an embodiment of the present invention was determined. The experiment was performed in the same manner as in Comparative Example 1, except that a silica chip having a pillar structure was used as a substrate, 2M of sodium sulfate(pH 4) was used as a kosmotropic salt, E. coli gDNA 1377ng was used as nucleic acid, and 10 mM of Tris-HCl(pH 9) was used as a nucleic acid eluting buffer. The nucleic acid eluting buffer has a similar composition to that of a general PCR buffer. Binding efficiency and eluting efficiency of nucleic acid was compared using a Qiagen solution as a control group.

FIG. 5 is a graph showing binding efficiency and eluting efficiency of E. coli gDNA using a kosmotropic salt. In FIG. 5, A refers to when SO₄²- is used as a kosmotropic salt, and B refers to when a Qiagen solution is used as a control group. As can be seen in FIG. 5, binding efficiency of nucleic acid in both a kosmotropic salt and Qiagen solution is very high at pH 4. However, the DNA recovery yield is higher in the case of using a kosmotropic salt according to an embodiment of the present invention than in the case of using a Qiagen solution in terms of eluting efficiency.

Therefore, when nucleic acid is purified using a kosmotropic salt, binding efficiency and eluting efficiency of nucleic acid are very high, so that the method according to an embodiment of the present invention can be efficiently used for nucleic acid purification.

According to the present invention, since a solid support can be used as it is without any surface treatment, manufacture of an apparatus for purifying nucleic acid on hydrophilic surface of a solid support is very easy, and nucleic acid can be bound to the solid support without specific additives in a wide pH range, so that the apparatus can be used for a Lab-On-a-Chip.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A method of purifying nucleic acid using a solid support which has a hydrophilic functional group on its surface, the method comprising:
contacting a nucleic acid-containing sample and a solution comprising a kosmotropic salt on the solid support to bind the nucleic acid to the solid support.

2. The method of claim 1, further comprising eluting the nucleic acid bound to the solid support by addition of water or a nucleic acid eluting buffer.

3. The method of claim 1, wherein the kosmotropic salt is selected from the group consisting of sulfate(SO₄²⁻), phosphate(HPO₄²⁻), hydroxide(OH⁻), fluoride(F⁻), formate(HCOO⁻) and acetate(CH₃COO⁻).

4. The method of claim 1, wherein the nucleic acid-containing sample and the solution containing a kosmotropic salt has a pH of 3 to 10.

5. The method of claim 1 or claim 4, wherein the concentration of the kosmotropic salt is 100~2000 mM.

6. The method of claim 1, wherein the solid support is selected from the group consisting of slide glass, a silicon wafer, a magnetic bead, polystyrene, a membrane and a metal plate.

7. The method of claim 2, wherein the nucleic acid eluting buffer is selected from the group consisting of phosphate, Tris, HEPES, CHES and borate.

8. The method of claim 2, wherein the nucleic acid eluting buffer has a pH of 5-10.

9. The method of claim 2, wherein the concentration of the nucleic acid eluting buffer is less than or equal to100 mM.

10. The method of claim 1, wherein the nucleic acid-containing sample is selected from the group consisting of blood, serum, urine, saliva, ocular lens fluid, cerebrospinal fluid, milk, ascite fluid, synovial fluid, peritoneal cavity liquid, amniotic fluid, tissue, fermentation broth, cell culture fluid, nucleic acid amplification reaction product and nucleic acid synthesis product.

11. The method of claim 2, further comprising detecting or amplifying the eluted nucleic acid after the nucleic acid is eluted from the solid support.

12. The method of claim 11, wherein the amplifying the nucleic acid is performed without removing the nucleic acid eluting buffer.

13. An apparatus for purifying nucleic acid, the apparatus comprising: a solid support having a hydrophilic functional group on its surface; and a kosmotropic salt solution storing part that is interconnected to the solid support through a microchannel and provides a kosmotropic salt to the solid support.

14. The apparatus of claim 13, further comprising a nucleic acid eluting buffer storing part that is interconnected to the solid support through a micro channel, and provides a nucleic acid eluting buffer to the solid support.

15. The apparatus of claim 13, wherein the solid support has a planar structure, a pillar structure, a bead structure or a sieve structure.

16. The apparatus of claim 13, wherein the solid support is selected from the group consisting of slide glass, a silicon wafer, a magnetic bead, polystyrene, a membrane and a metal plate.

17. The apparatus of claim 13, wherein the kosmotropic salt is selected from the group consisting of sulfate(SO₄²⁻), phosphate(HPO₄²⁻), hydroxide(OH⁻), fluoride(F⁻), formate(HCOO⁻) and acetate(CH₃COO⁻).

18. The apparatus of claim 13, further comprising a nucleic acid amplification part or a nucleic acid detection part.

19. A lab-on-a-chip comprising the apparatus for purifying nucleic acid of claim 13.
